(19) Europäisches Patentamt
European Patent Office
Office européen des brevets

(11) **EP 4 501 413 A1**

(12) **EUROPEAN PATENT APPLICATION**
published in accordance with Art. 153(4) EPC

(43) Date of publication:
**05.02.2025 Bulletin 2025/06**

(21) Application number: **23774290.3**

(22) Date of filing: **10.02.2023**

(51) International Patent Classification (IPC):
**A61Q 5/02** (2006.01)        **A61K 8/46** (2006.01)

(52) Cooperative Patent Classification (CPC):
**A61K 8/46; A61Q 5/02**

(86) International application number:
**PCT/JP2023/004500**

(87) International publication number:
**WO 2023/181691 (28.09.2023 Gazette 2023/39)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **24.03.2022 JP 2022048621**

(71) Applicant: **Kao Corporation
Tokyo 103-8210 (JP)**

(72) Inventor: **MIYAGAWA, Takuya
Tokyo 131-8501 (JP)**

(74) Representative: **Hoffmann Eitle
Patent- und Rechtsanwälte PartmbB
Arabellastraße 30
81925 München (DE)**

(54) **HAIR WASHING COMPOSITION**

(57) The present invention relates to a hair cleansing composition containing an aromatic sulfonic acid or a salt thereof and, when applied to hair, exhibits superior characteristics from the time of cleansing to the time of rinsing and after cleansing. That is, the present invention is a hair cleansing composition containing the following components (A) and (B): (A) 1 mass% or more and 8 mass% or less of an aromatic sulfonic acid or a salt thereof, and (B) 4 mass% or more and 25 mass% or less of an internal olefin sulfonic acid having 16 carbon atoms or a salt thereof obtained by sulfonation of a raw material olefin having 16 carbon atoms and having an average double bond position of 3.9-position or more and 4.4-position or less, wherein the hair cleansing composition has a pH at 25°C of 1 or more and less than 5 when formed into a 5 mass% aqueous solution.

EP 4 501 413 A1

# EP 4 501 413 A1

## Description

### Field of the Invention

The present invention relates to a hair cleansing composition.

### Background of the Invention

Aromatic sulfonic acids or salts thereof which are known to be capable of imparting the desired effects have been used in many compositions to be applied to hair.

For example, Patent Literature 1 discloses a foam composition containing sodium xylensulfonate as a viscosity regulator and containing an anionic surfactant and a foaming agent such as propane or isobutan each in a specific amount, and the density of foam is regulated to a specific range to suitably deliver effective amounts of active substances as a haircare composition. Patent Literature 2 discloses a composition containing an aromatic sulfonic acid compound such as sulfanylic acid having an amino group or the like, or a salt thereof, and an attempt is made to remedy frizzed keratin fiber such as hair or straighten it, and impart manageability and smoothness to keratin fiber.

Meanwhile, warping or frizzing of hair resulting from stimuli such as ultraviolet rays and dryer heat to hair, deactivation of the scalp or the change of follicles due to aging, or the like can cause persistent curls and waves of hair. In recent years, it is becoming increasingly and strongly desirable to reduce such curls and waves of hair and achieve straight, glossy, manageable, and lively hair.

### Citation List

### Patent Literatures

   Patent Literature 1: JP-A-2019-505546
   Patent Literature 2: JP-A-2019-73443

### Summary of the Invention

The present invention provides a hair cleansing composition containing the following components (A) and (B):

   (A) 1 mass% or more and 8 mass% or less of an aromatic sulfonic acid or a salt thereof, and
   (B) 4 mass% or more and 25 mass% or less of an internal olefin sulfonic acid having 16 carbon atoms or a salt thereof obtained by sulfonation of a raw material olefin having 16 carbon atoms and having an average double bond position of 3.9-position or more and 4.4-position or less,

wherein the hair cleansing composition has a pH at 25°C of 1 or more and less than 5 when formed into a 5 mass% aqueous solution.

Patent Literature 1 is not at all concerned with reducing curls and waves in hair and, accordingly, there is sufficient room for improvement. Also, even with the technique set forth in Patent Literature 2, a certain amount of time is required to obtain a sufficient effect. Accordingly, repetitive use on a daily basis still places an excessive burden on the user. Thus, a hair cleansing agent capable of sufficiently reducing curls and waves in hair while reducing the burden on the user is not yet achieved.

The present invention relates to a hair cleansing composition containing an aromatic sulfonic acid or a salt thereof and, when applied to hair, exhibits superior characteristics from the time of cleansing to the time of rinsing and after cleansing.

The present inventor have conducted various studies and found that a hair cleansing composition which is capable of effectively reducing curls and waves in hair even when the duration of use per time is relatively short can be obtained by containing an aromatic sulfonic acid or a salt thereof and a specific internal olefinsulfonic acid or a salt thereof each in a specific amount and regulating pH to a specific range.

The hair cleansing composition of the present invention is capable of effectively and promptly reducing curls and waves in hair while making it possible to reduce the duration of use required per time. Also, the hair cleansing composition is capable of providing a sufficient volume of foam as a hair cleaning agent and reducing friction of hair during rinsing after cleansing. Accordingly, the hair cleansing composition is capable of readily achieving straight, glossy, manageable, and lively hair in daily repetitive use while effectively reducing the burden on the user.

Detailed Description of the Invention

The present invention will now be described in detail.

The hair cleansing composition of the present invention contains 1 mass% or more and 8 mass% or less of an aromatic sulfonic acid or a salt thereof as a component (A) . The component (A) enables the effect of reducing curls and waves in hair to be effectively and promptly exhibited.

Specific examples of the component (A) include one or more selected from the group consisting of benzenesulfonic acid or a salt thereof, naphthalenesulfonic acid or a salt thereof, azulenesulfonic acid or a salt thereof, and benzophenonesulfonic acid or a salt thereof. Examples of such salts of the component (A) include a sodium salt, a potassium salt, a lithium salt, an aluminum salt, an ammonium salt ($NH^{4+}$), and an organoammonium salt.

More specific examples of benzenesulfonic acid or a salt thereof include one or more selected from the group consisting of benzenesulfonic acid, o-toluenesulfonic acid, p-toluenesulfonic acid, xylenesulfonic acid, cumenesulfonic acid, ethylbenzenesulfonic acid, 2,4,6-trimethylbenzenesulfonic acid, and salts thereof.

More specific examples of naphthalenesulfonic acid or a salt thereof include one or more selected from the group consisting of 1- or 2-naphthalenesulfonic acid ($\alpha$- or $\beta$-naphthalenesulfonic acid), 2,7-naphthalenedisulfonic acid, 1,5-naphthalenedisulfonic acid, 2,6-naphthalenedisulfonic acid, 1-naphthol-2-sulfonic acid, 1-naphthol-4-sulfonic acid, 2-naphthol-6-sulfonic acid, 2-naphthol-7-sulfonic acid, 2,3-dihydroxynaphthalene-6-sulfonic acid, 1,7-dihydroxynaphthalene-3-sulfonic acid, J acid (2-amino-5-naphthol-7-sulfonic acid), 1-amino-2-naphthol-4-sulfonic acid, 1-naphthylamine-4-sulfonic acid, Broenner's acid (2-naphthylamine-6-sulfonic acid), Cleve's acid (1-naphthylamine-7-sulfonic acid), 2-naphthylamine-1-sulfonic acid, 1-naphthylamine-6-sulfonic acid, 1-naphthylamine-8-sulfonic acid, 2,7-diamino-1-naphthol-3-sulfonic acid, 7,8-diamino-1-naphthol-3-sulfonic acid, 6-methyl-2-naphthalenesulfonic acid, 4-ethyl-1-naphthalenesulfonic acid, 5-isopropyl-1-naphthalenesulfonic acid, 5-butyl-2-naphthalenesulfonic acid, and salts thereof.

More specific examples of azulenesulfonic acid or a salt thereof include one or more selected from the group consisting of guaiazulenesulfonic acid, 1-azulenesulfonic acid, 3-acetyl-7-isopropyl-1-azulenesulfonic acid, 3-(2-hydroxyethyl)-7-isopropyl-1-azulenesulfonic acid, 3-methyl-7-isopropyl-1-azulenesulfonic acid, 7-isopropyl-1-azulenesulfonic acid, 1,4-dimethyl-7-isopropyl-2-azulenesulfonic acid, 1,3-azulenedisulfonic acid, 3-formyl-4,6,8-trimethyl-1-azulenesulfonic acid, and salts thereof.

More specific examples of benzophenonesulfonic acid or a salt thereof include one or more selected from the group consisting of o-chlorobenzophenonesulfonic acid, p-chlorobenzophenonesulfonic acid, 2-hydroxybenzophenonesulfonic acid, 4-hydroxybenzophenonesulfonic acid, 2-aminobenzophenonesulfonic acid, 4-aminobenzophenonesulfonic acid, 2-methylbenzophenonesulfonic acid, 4-methoxybenzophenonesulfonic acid, 4,4'-dimethylbenzophenonesulfonic acid, and salts thereof.

Among these components (A), from the viewpoint of synergistically exhibiting the effect of reducing curls and waves in hair together with the component (B) described below, one or more selected from the group consisting of benzenesulfonic acid, naphthalenesulfonic acid, and salts thereof are preferable, and one or more selected from the group consisting of o-toluenesulfonic acid, p-toluenesulfonic acid, 2-naphthalenesulfonic acid, and salts thereof are more preferable.

The content of the component (A) in the hair cleansing composition of the present invention is 1 mass% or more, preferably 1.5 mass% or more, and more preferably 2 mass% or more, and is 8 mass% or less, preferably 6 mass% or less, and more preferably 4 mass% or less, from the viewpoint of effectively and promptly exhibiting the effect of reducing curls and waves in hair. The content of the component (A) in the hair cleansing composition of the present invention is 1 mass% or more and 8 mass% or less, preferably from 1.5 to 6 mass%, and more preferably from 2 to 4 mass%.

The hair cleansing composition of the present invention contains, as a component (B), 4 mass% or more and 25 mass% or less of an internal olefin sulfonic acid having 16 carbon atoms or a salt thereof obtained by sulfonation of a raw material olefin having 16 carbon atoms and having an average double bond position of 3.9-position or more and 4.4-position or less. That is, the internal olefin sulfonic acid having 16 carbon atoms or a salt thereof as the component (B) is a compound that is obtained by using a raw material olefin having an average double bond position within a specific limited range as a starting raw material and sulfonating it, specifically, a compound that is obtained by sulfonating a raw material olefin and then neutralizing and hydrolyzing it.

Examples of the salt of the internal olefin sulfonic acid having 16 carbon atoms obtained by sulfonation of a raw material olefin having 16 carbon atoms and having an average double bond position of 3.9-position or more and 4.4-position or less include one or more selected from the group consisting of alkali metal salts, such as a sodium salt and a potassium salt; organic amine salts, such as an ammonium salt, a monoethanolamine salt, a diethanolamine salt, a triethanolamine salt, a 2-aminoethanol salt, and a 2-aminomethyl propanediol salt; and basic amino acid salts, such as a lysine salt and an arginine salt. These internal olefin sulfonates having 16 carbon atoms may not be necessarily in a salt form from the beginning and may be a salt that is generated by a neutralization reaction during manufacturing.

In particular, the salt of the internal olefin sulfonic acid having 16 carbon atoms is, from the viewpoint of effectively enhancing the effect of reducing curls and waves in hair together with the component (A), preferably one or more selected from the group consisting of a sodium salt, a potassium salt, an ammonium salt, and a 2-aminoethanol salt, more preferably one or two selected from the group consisting of a sodium salt and a potassium salt, and further more preferably a sodium salt. That is, sodium internal olefin sulfonate having 16 carbon atoms is further more preferable.

The internal olefin sulfonic acid having 16 carbon atoms or a salt thereof as the component (B), which is a product obtained from such a raw material olefin having 16 carbon atoms, is mainly a mixture of hydroxyalkanesulfonic acid having 16 carbon atoms or a salt thereof (hydroxy form, abbreviation: "HAS") and olefin sulfonic acid having 16 carbon atoms or a salt thereof (olefin form, abbreviation: "IOS").

In the raw material olefin having 16 carbon atoms as a starting raw material of the component (B), the positions of double bonds are mainly located inside the carbon chain, but a so-called α-olefin in which the double bond is located at the 1-position of the carbon chain may be contained in a trace amount. When such a raw material olefin is sulfonated, β-sultone is mainly produced, and a part of the β-sultone is converted into γ-sultone and an olefin sulfonic acid. These β-sultone, γ-sultone, and olefin sulfonic acid are further converted into hydroxyalkanesulfonic acid having 16 carbon atoms or a salt thereof and olefin sulfonic acid having 16 carbon atoms or a salt thereof in the neutralization/hydrolysis process (for example, J. Am. Oil Chem. Soc. 69, 39 (1992)). The hydroxy group of the resulting hydroxyalkanesulfonic acid or a salt thereof is located inside the alkane chain, and the double bond of the olefin sulfonic acid or a salt thereof is located inside the olefin chain.

Accordingly, in the present specification, these products and a mixture thereof are collectively referred to as an internal olefin sulfonic acid having 16 carbon atoms or a salt thereof as the component (B).

The raw material olefin having 16 carbon atoms that is sulfonated and forms an internal olefin sulfonic acid having 16 carbon atoms or a salt thereof as the component (B) has an average double bond position of 3.9-position or more and 4.4-position or less. The raw material olefin having 16 carbon atoms and having an average double bond position of such a value has a broad distribution of double bond positions over from the 2-position to the 8-position including the 1-position that can be contained in a trace amount.

The double bond positions and its distribution in a raw material olefin can be verified by measurement using a gas chromatography mass spectrometer (abbreviation: "GC-MS"). Specifically, each of components having different carbon chain lengths and double bond positions is precisely separated by a gas chromatography apparatus (hereinafter, abbreviated as GC), and the double bond positions can be identified by application of them to a mass spectrometer (abbreviation: "MS"), and the distribution is determined from the respective GC peak areas.

On the other hand, in the internal olefin sulfonic acid having 16 carbon atoms or a salt thereof as the component (B) obtained by sulfonation of such a raw material olefin, the more the position of the sulfonate group introduced by sulfonation is inside the carbon chain, the more difficult the separation. Accordingly, there is no definitive analytical method at present. However, the positions of the sulfonate groups in the component (B) are fully presumed to approximately correspond to the double bond positions in the raw material olefin and to show a broad distribution over from the 2-position to the 8-position including the 1-position without being excessively unevenly distributed. Accordingly, in the present invention, the component (B) is regulated based on the value of the average double bond position in the raw material olefin as a starting raw material.

The component (B) used in the present invention is an internal olefin sulfonic acid or a salt thereof obtained from a raw material olefin having the above-mentioned average double bond position value, i.e., a broad double bond distribution, and the sulfonate groups in the carbon chain are present in broad positions without being excessively unevenly distributed. In the internal olefin sulfonic acid having 16 carbon atoms or a salt thereof, when sulfonic acid groups are unevenly distributed more toward the carbon chain terminals, the melting point is increased due to the increased elongated carbon chains, possibly resulting in precipitation. In contrast, if sulfonate groups are unevenly distributed near the interior of the carbon chain, the foam quality at the time of cleansing and the feel of hair at the time of rinsing are deteriorated, and the hair cleansing effect may be reduced. However, in the internal olefin sulfonic acid having 16 carbon atoms or a salt thereof as the component (B) obtained from the above-mentioned raw material olefin, sulfonate groups are present in broad positions without being excessively unevenly distributed in the carbon chain, the internal olefin sulfonic acids or salts thereof having various lengths of the carbon chain from the bonding position of the sulfonate group to the end are moderately mixed. Accordingly, the component (B), in cooperation with the component (A), can exhibit the excellent hair cleansing effect from the time of cleansing to the time of rinsing and after cleansing. The same applies also when the component (B) is sodium internal olefin sulfonate having 16 carbon atoms.

The average double bond position (unit: position, abbreviated as "DBP") in the raw material olefin having 16 carbon atoms as the starting raw material of the component (B) means the average value of the double bond positions of each raw material olefin having 16 carbon atoms present in the total amount of the raw material olefin having 16 carbon atoms. Specifically, the average double bond position of a raw material olefin having 16 carbon atoms is a value determined by the following expression (1).

$$\text{Average double bond position of raw material olefin having 16 carbon atoms} = \sum_{n=1}^{8} n \times C_n / 100 \qquad \cdots (1)$$

(In the expression (1), n represents an integer (unit: position) indicating the position of a double bond existing in a raw material olefin having 16 carbon atoms; and Cn represents the content (unit: mass%) of the raw material olefin having 16 carbon atoms and having a double bond at n-position in the total amount of 100 mass% of the raw material olefin having 16 carbon atoms.)

The average double bond position in the raw material olefin having 16 carbon atoms forming the component (B) is, from the viewpoint of securing the exhibition of excellent hair cleansing effect, 3.9-position or more and preferably 4.0-position or more, and is 4.4-position or less, preferably 4.3-position or less, and more preferably 4.2-position or less. In addition, the average double bond position in the raw material olefin having 16 carbon atoms is 3.9-position or more and 4.4-position or less, preferably from 4.0-position to 4.4-position, more preferably from 4.0-position to 4.3-position, and further more preferably from 4.0-position to 4.2-position.

In the raw material olefin having 16 carbon atoms, the content of a raw material olefin having a double bond position at the 2-position in the raw material olefin having 16 carbon atoms is preferably 10 mass% or more, more preferably 15 mass% or more, and further more preferably 20 mass% or more and preferably 35 mass% or less, more preferably 32 mass% or less, and further more preferably 24 mass% or less. In addition, in the raw material olefin having 16 carbon atoms, the content of the raw material olefin having a double bond position at the 2-position in the raw material olefin having 16 carbon atoms is preferably from 10 to 35 mass%, more preferably from 15 to 32 mass%, and further more preferably from 20 to 24 mass%.

In the raw material olefin having 16 carbon atoms, the content of a raw material olefin having a double bond position at the 3-position in the raw material olefin having 16 carbon atoms is preferably 10 mass% or more, more preferably 14 mass% or more, and further more preferably 16 mass% or more and preferably 30 mass% or less, more preferably 24 mass% or less, and further more preferably 19 mass% or less. In addition, in the raw material olefin having 16 carbon atoms, the content of the raw material olefin having a double bond position at the 3-position in the raw material olefin having 16 carbon atoms is preferably from 10 to 30 mass%, more preferably from 14 to 24 mass%, and further more preferably from 16 to 19 mass%.

In the raw material olefin having 16 carbon atoms, the content of a raw material olefin having a double bond position at the 4-position in the raw material olefin having 16 carbon atoms is preferably 10 mass% or more, more preferably 15 mass% or more, and further more preferably 17 mass% or more and preferably 30 mass% or less, more preferably 25 mass% or less, and further more preferably 19 mass% or less. In addition, in the raw material olefin having 16 carbon atoms, the content of the raw material olefin having a double bond position at the 4-position in the raw material olefin having 16 carbon atoms is preferably from 10 to 30 mass%, more preferably from 15 to 25 mass%, and further more preferably from 17 to 19 mass%.

In the raw material olefin having 16 carbon atoms, the content of a raw material olefin having a double bond position at the 5-position in the raw material olefin having 16 carbon atoms is preferably 5 mass% or more, more preferably 10 mass% or more, and further more preferably 13 mass% or more and preferably 25 mass% or less, more preferably 19 mass% or less, and further more preferably 15 mass% or less. In addition, in the raw material olefin having 16 carbon atoms, the content of the raw material olefin having a double bond position at the 5-position is preferably from 5 to 25 mass%, more preferably from 10 to 19 mass%, and further more preferably from 13 to 15 mass% in the raw material olefin having 16 carbon atoms.

In the raw material olefin having 16 carbon atoms, the content of a raw material olefin having a double bond position at the 6-position in the raw material olefin having 16 carbon atoms is preferably 5 mass% or more, more preferably 7 mass% or more, and further more preferably 11 mass% or more and preferably 20 mass% or less, more preferably 15 mass% or less, and further more preferably 13 mass% or less. In addition, in the raw material olefin having 16 carbon atoms, the content of the raw material olefin having a double bond position at the 6-position in the raw material olefin having 16 carbon atoms is preferably from 5 to 20 mass%, more preferably from 7 to 15 mass%, and further more preferably from 11 to 13 mass%.

In the raw material olefin having 16 carbon atoms, the total content of raw material olefins having a double bond position at the 7-position or 8-position in the raw material olefin having 16 carbon atoms is preferably 5 mass% or more, more preferably 7 mass% or more, and further more preferably 12 mass% or more and preferably 25 mass% or less, more preferably 22 mass% or less, and further more preferably 16 mass% or less. In addition, in the raw material olefin having 16 carbon atoms, the total content of the raw material olefins having a double bond position at the 7-position or 8-position in the raw material olefin having 16 carbon atoms is preferably from 5 to 25 mass%, more preferably from 7 to 22 mass%, and further more preferably from 12 to 16 mass%.

In the raw material olefin having 16 carbon atoms, the mass ratio of the content of raw material olefins having a double bond position at the 3- to 5-positions to the content of raw material olefins having a double bond position at the 6- to 8-positions, (raw material olefin 3 to 5-position/raw material olefin 6 to 8-position), is preferably 1.0 or more, more preferably 1.3 or more, and further more preferably 1.7 or more and preferably 4.0 or less, more preferably 3.5 or less, and further more preferably 2.2 or less. In addition, in the raw material olefin having 16 carbon atoms, the mass ratio of the content of raw material olefins having a double bond position at the 3- to 5-positions to the content of raw material olefins having a double bond position at the 6- to 8-positions, (raw material olefin $_{3\ to\ 5}$-position/raw material olefin $_{6\ to\ 8}$-position), is preferably from 1.0 to 4.0, more preferably from 1.3 to 3.5, and further more preferably from 1.7 to 2.2.

In the raw material olefin having 16 carbon atoms, the content of a raw material olefin having a double bond position at the 1-position ($\alpha$-olefin), which may inevitably exist, is preferably less than 5.0 mass%, more preferably less than 3.0 mass%, and further more preferably less than 2.5 mass% in the raw material olefin having 16 carbon atoms. Alternatively, the raw material olefin having 16 carbon atoms preferably does not contain $\alpha$-olefin.

The above-mentioned raw material olefin having 16 carbon atoms can be obtained by isomerization (double bond transfer) of a raw material olefin having a double bond position at the 1-position ($\alpha$-olefin) that is generated by dehydration reaction of an alcohol having 16 carbon atoms. Specifically, a solid acid catalyst, such as alumina, in an amount of preferably 0.5 parts by mass or more, more preferably 2 parts by mass or more, and preferably 15 parts by mass or less, more preferably 10 parts by mass or less, and preferably from 0.5 to 15 parts by mass, more preferably from 2 to 10 parts by mass, is added to 100 parts by mass of 1-hexadecanol.

Subsequently, isomerization reaction is performed by stirring at preferably 220°C or more, more preferably 260°C or more, and preferably 350°C or less, and preferably from 220°C to 350°C, more preferably from 260°C to 350°C, for preferably 1 hour or more, more preferably 3 hours or more, and preferably 30 hours or less, more preferably 10 hours or less, and preferably from 1 to 30 hours, more preferably from 3 to 10 hours. The product after completion of the reaction is appropriately distilled to obtain above-mentioned raw material olefin having 16 carbon atoms.

In an internal olefin sulfonic acid having 16 carbon atoms or a salt thereof as the component (B) obtained by sulfonation of the above-described raw material olefin having 16 carbon atoms, the content of the internal olefin sulfonic acid having a sulfonate group at the 1-position or more and the 4-position or less or a salt thereof in the component (B) is preferably 40 mass% or more, more preferably 50 mass% or more, and further more preferably 55 mass% or more and preferably 75 mass% or less, more preferably 70 mass% or less, and further more preferably 68 mass% or less. In addition, in the internal olefin sulfonic acid having 16 carbon atoms or a salt thereof as the component (B) obtained by sulfonation of the above-described raw material olefin having 16 carbon atoms, the content of the internal olefin sulfonic acid having a sulfonate group at the 1-position or more and the 4-position or less or a salt thereof in the component (B) is preferably 40 mass% or more and 75 mass% or less, more preferably from 50 to 70 mass%, and further more preferably from 55 to 68 mass%.

When the component (B) is sodium internal olefin sulfonate having 16 carbon atoms, the content of sodium internal olefin sulfonate having a sulfonate group at the 1-position or more and 4-position or less in the sodium internal olefin sulfonate having 16 carbon atoms is also same as above.

In the internal olefin sulfonic acid having 16 carbon atoms or a salt thereof as the component (B), the content of an internal olefin sulfonic acid having a sulfonate group at the 2-position or a salt thereof in the component (B) is preferably 10 mass% or more, more preferably 13 mass% or more, and further more preferably 17 mass% or more and preferably 35 mass% or less, more preferably 30 mass% or less, and further more preferably 25 mass% or less. In addition, in the internal olefin sulfonic acid having 16 carbon atoms or a salt thereof as the component (B), the content of the internal olefin sulfonic acid having a sulfonate group at the 2-position or a salt thereof in the component (B) is preferably 10 mass% or more and 35 mass% or less, more preferably from 13 to 30 mass%, and further more preferably from 17 to 25 mass%.

When the component (B) is sodium internal olefin sulfonate having 16 carbon atoms, in the sodium internal olefin sulfonate having 16 carbon atoms, the content of sodium internal olefin sulfonate having a sulfonate group at the 2-position is also same as above.

In the internal olefin sulfonic acid having 16 carbon atoms or a salt thereof as the component (B), the content of an internal olefin sulfonic acid having a sulfonate group at the 3-position or a salt thereof in the component (B) is preferably 5 mass% or more, more preferably 11 mass% or more, and further more preferably 15 mass% or more and preferably 30 mass% or less, more preferably 25 mass% or less, and further more preferably 20 mass% or less. In addition, in the internal olefin sulfonic acid having 16 carbon atoms or a salt thereof as the component (B), the content of the internal olefin sulfonic acid having a sulfonate group at the 3-position or a salt thereof in the component (B) is preferably 5 mass% or more and 30 mass% or less, more preferably from 11 to 25 mass%, and further more preferably from 15 to 20 mass%.

When the component (B) is sodium internal olefin sulfonate having 16 carbon atoms, the content of sodium internal olefin sulfonate having a sulfonate group at the 3-position in the sodium internal olefin sulfonate having 16 carbon atoms is also same as above.

In the internal olefin sulfonic acid having 16 carbon atoms or a salt thereof as the component (B), the content of an internal olefin sulfonic acid having a sulfonate group at the 4-position or a salt thereof in the component (B) is preferably 15 mass% or more, more preferably 18 mass% or more, and further more preferably 19 mass% or more and preferably 30 mass% or less, more preferably 25 mass% or less, and further more preferably 23 mass% or less. In addition, in the internal olefin sulfonic acid having 16 carbon atoms or a salt thereof as the component (B), the content of the internal olefin sulfonic acid having a sulfonate group at the 4-position or a salt thereof in the component (B) is preferably 15 mass% or more and 30 mass% or less, more preferably from 18 to 25 mass%, and further more preferably from 19 to 23 mass%.

When the component (B) is sodium internal olefin sulfonate having 16 carbon atoms, in the sodium internal olefin sulfonate having 16 carbon atoms, the content of sodium internal olefin sulfonate having a sulfonate group at the 4-position is also same as above.

In the internal olefin sulfonic acid having 16 carbon atoms or a salt thereof as the component (B), the content of an internal olefin sulfonic acid having a sulfonate group at the 1-position or a salt thereof in the component (B) is preferably less than 5.0 mass%, more preferably less than 3.0 mass%, and further more preferably less than 2.5 mass%. Alternatively, the internal olefin sulfonic acid having 16 carbon atoms or a salt thereof as the component (B) preferably does not contain the internal olefin sulfonic acid having a sulfonate group at the 1-position or a salt thereof.

When the component (B) is sodium internal olefin sulfonate having 16 carbon atoms, the content of sodium internal olefin sulfonate having a sulfonate group at the 1-position in the sodium internal olefin sulfonate having 16 carbon atoms is also same as above. Alternatively, it is preferable that the sodium internal olefin sulfonate having a sulfonate group at the 1-position is not contained.

In the internal olefin sulfonic acid having 16 carbon atoms or a salt thereof as the component (B), the mass ratio of the content of the hydroxy form (HAS) to the content of the olefin form (IOS), hydroxy form/olefin form, is preferably from 50/50 to 100/0, more preferably from 60/40 to 100/0, further more preferably from 70/30 to 100/0, further more preferably from 75/25 to 100/0, and more preferably from 75/25 to 95/5 from the viewpoint of productivity improvement and impurity reduction.

Such a mass ratio (hydroxy form/olefin form) is determined based on HPLC-MS peak areas that are obtained by separation of the hydroxy form and the olefin form from the component (B) by HPLC and application to MS.

When the component (B) is sodium internal olefin sulfonate having 16 carbon atoms, the mass ratio of the content of the hydroxy form (HAS) to the content of the olefin form (IOS), hydroxy form/olefin form, in the sodium internal olefin sulfonate having 16 carbon atoms is also same as above.

Since the internal olefin sulfonic acid having 16 carbon atoms or a salt thereof as the component (B) can be obtained by sulfonation of a raw material olefin, there is a possibility that the unreacted raw material olefin and the inorganic compound remain in the component (B). Lower contents of these components are preferable. The same applies also when the component (B) is sodium internal olefin sulfonate having 16 carbon atoms.

In the internal olefin sulfonic acid having 16 carbon atoms or a salt thereof as the component (B), the content of the unreacted raw material olefin in the component (B) is preferably less than 5.0 mass%, more preferably less than 3.0 mass%, further more preferably less than 1.5 mass%, and further more preferably less than 1.0 mass%.

When the component (B) is sodium internal olefin sulfonate having 16 carbon atoms, the content of the unreacted raw material olefin in the sodium internal olefin sulfonate having 16 carbon atoms is also same as above.

In the internal olefin sulfonic acid having 16 carbon atoms or a salt thereof as the component (B), the content of the inorganic compound in the component (B) is preferably less than 7.5 mass%, more preferably less than 5.0 mass%, further more preferably less than 3.0 mass%, further more preferably less than 2.0 mass%, and even more preferably less than 1.6 mass%.

When the component (B) is sodium internal olefin sulfonate having 16 carbon atoms, the content of the inorganic compound in the sodium internal olefin sulfonate having 16 carbon atoms is also same as above.

The content of the component (B) in the hair cleansing composition of the present invention is 4 mass% or more, and is 25 mass% or less, preferably 20 mass% or less, more preferably 18 mass% or less, and further more preferably 16 mass% or less, from the viewpoint of effectively and promptly exhibiting the effect of reducing curls and waves in hair. In addition, the content of the component (B) in the hair cleansing composition of the present invention is 4 mass% or more and 25 mass% or less, preferably from 4 to 20 mass%, more preferably from 4 to 18 mass%, and further more preferably from 4 to 16 mass%.

The total content of the content of the component (A) and the content of the component (B), (A)+(B), in the hair cleansing composition of the present invention is preferably 5 mass% or more, more preferably 8 mass% or more, and further more preferably 10 mass% or more, and is preferably 30 mass% or less, more preferably 25 mass% or less, and further more preferably 20 mass% or less, from the viewpoint of more effectively and promptly exhibiting the effect of reducing curls and waves in hair. In addition, the total content of the content of the component (A) and the content of the component (B), (A)+(B), in the hair cleansing composition of the present invention is preferably from 5 to 30 mass%, more preferably from 8 to 25 mass%, and further more preferably from 10 to 20 mass%.

The mass ratio of the content of the component (A) to the content of the component (B), (A)/(B), is preferably 0.05 or more, more preferably 0.08 or more, further more preferably 0.13 or more, and even more preferably 0.15 or more, and is preferably 2 or less, more preferably 1.5 or less, further more preferably 1.0 or less, and even more preferably 0.8 or less, from the viewpoint of more effectively and promptly exhibiting the effect of reducing curls and waves in hair, from the viewpoint of providing a sufficient volume of foam as a hair cleaning agent, and from the viewpoint of reducing friction of hair during rinsing after cleansing. In addition, the mass ratio of the content of the component (A) to the content of the component (B), (A)/(B), is preferably 0.05 or more and 2 or less, more preferably from 0.08 to 1.5, further more preferably from 0.13 to 1.0, and even more preferably from 0.15 to 0.8.

The internal olefin sulfonic acid having 16 carbon atoms or a salt thereof as the component (B) can be obtained through sulfonation by reacting the raw material olefin having 16 carbon atoms with sulfur trioxide, specifically, by sulfonating the raw material olefin and then performing neutralization and subsequently hydrolysis.

More specifically, the amount of the sulfur trioxide that is used for sulfonation of the raw material olefin is preferably 0.8 mol or more, more preferably 0.9 mol or more, and further more preferably 0.95 mol or more based on 1 mol of the raw material olefin from the viewpoint of improving the yield of the component (B) and the viewpoint of improving the reactivity. In addition, the amount of the sulfur trioxide that is used for sulfonation of the raw material olefin is preferably 1.2 mol or less, more preferably 1.1 mol or less, and further more preferably 1.05 mol or less from the viewpoint of the economic efficiency and the viewpoint of suppressing unnecessary coloring of the component (B). In addition, the amount of the sulfur trioxide that is used for sulfonation of the raw material olefin is preferably from 0.8 to 1.2 mol, more preferably from 0.9 to 1.1 mol, and further more preferably from 0.95 to 1.05 mol based on 1 mol of the raw material olefin.

The reaction temperature when the raw material olefin is sulfonated is preferably 0°C or more from the viewpoint of preventing coagulation of the sulfur trioxide and the component (B) and is preferably 50°C or less from the viewpoint of suppressing unnecessary coloring of the component (B). The reaction temperature when the raw material olefin is sulfonated is preferably from 0°C to 50°C.

In the neutralization, an alkali compound, such as sodium hydroxide, potassium hydroxide, ammonia, and 2-aminoethanol, is used for the reaction. The amount of the alkali compound to be added is preferably 1.0 times mol or more, more preferably 1.03 times mol or more, per 1 mol of the sulfonate group from the viewpoint of suppressing the generation of impurities such as a raw material olefin and an inorganic salt and from the viewpoint of improving the reactivity. In addition, the amount of the alkali compound to be added is preferably 2.5 times mol or less, more preferably 2.0 times mol or less, and further more preferably 1.5 times mol or less per 1 mol of the sulfonate group from the viewpoint of the economic efficiency and the viewpoint of suppressing the generation of impurities such as a raw material olefin and an inorganic salt. The amount of the alkali compound to be added is preferably from 1.0 to 2.5 times mol, more preferably from 1.03 to 2.0 times mol, and further more preferably from 1.03 to 1.5 times mol per 1 mol of the sulfonate group.

In the neutralization, the temperature when the sulfonated raw material olefin and an alkali compound are mixed and the reaction temperature are preferably 40°C or less, more preferably 35°C or less, further more preferably 30°C or less, and further more preferably 25°C or less from the viewpoint of suppressing the generation of impurities, such as an internal olefin and an inorganic salt, by side reaction and are preferably 0°C or more, more preferably 10°C or more, further more preferably 15°C or more, and further more preferably 20°C or more from the viewpoint of improving the reactivity. In addition, the temperature when the sulfonated raw material olefin and an alkali compound are mixed and the reaction temperature are preferably from 0°C to 40°C, more preferably from 10°C to 35°C, further more preferably from 15°C to 30°C, and further more preferably from 20°C to 25°C.

The reaction temperature for hydrolysis that is performed after neutralization is preferably 120°C or more, more preferably 140°C or more, and further more preferably 160°C or more from the viewpoint of improving the reactivity in the presence of water. In addition, the reaction temperature for the hydrolysis is preferably 220°C or less and more preferably 180°C or less from the viewpoint of suppressing decomposition of the product. In addition, the reaction temperature for the hydrolysis is preferably from 120°C to 220°C, more preferably from 140°C to 180°C, and further more preferably from 160°C to 180°C.

The reaction time for the hydrolysis is preferably 30 minutes or more and more preferably 45 minutes or more from the viewpoint of completing the reaction. The reaction time for the hydrolysis is preferably 240 minutes or less, more preferably 180 minutes or less, further more preferably 120 minutes or less, and further more preferably 90 minutes or less from the viewpoint of improving the productivity. In addition, the reaction time for the hydrolysis is preferably from 30 to 240 minutes, more preferably from 45 to 180 minutes, further more preferably from 45 to 120 minutes, and further more preferably from 45 to 90 minutes. These reactions can be performed in series. After the completion of the reaction, purification can be performed by extraction, cleansing, and so on.

The same applies also when sodium internal olefin sulfonate having 16 carbon atoms is obtained as the component (B).

The hair cleansing composition of the present invention may further contain one or more selected from the group consisting of fatty acid polyoxyethylene sorbitan esters and polyoxyethylene alkyl ethers. Accordingly, the effect of reducing curls and waves in hair can be effectively and promptly exhibited, and also the volume of foam when the hair cleaning agent is applied is increased, enabling friction of hair during rinsing to be reduced as well.

The number of carbon atoms in the fatty acid residue of the fatty acid polyoxyethylene sorbitan ester is preferably 8 or more and more preferably 10 or more, and is preferably 18 or less and more preferably 14 or less. In the fatty acid polyoxyethylene sorbitan ester, the average number of moles of ethyleneoxy groups added is preferably 5 or more and more preferably 10 or more, and is preferably 50 or less and more preferably 30 or less.

Polyoxyethylene alkyl ether, as represented by the following general formula (2), can be used:

$$R^1\text{-O-(CH}_2\text{CH}_2\text{-O})_m\text{-H} \ldots \qquad (2)$$

wherein $R^1$ represents a linear or branched, saturated or unsaturated hydrocarbon group having 8 or more and 18 or fewer carbon atoms, and m represents an average value of from 5 to 50.

In the general formula (2), the number of carbon atoms of $R^1$ is preferably 10 or more and more preferably 12 or more, and is preferably 16 or less and more preferably 14 or less, from the viewpoint of effectively and promptly exhibiting the effect of reducing curls and waves in hair.

Specific examples of fatty acid polyoxyethylene sorbitan esters include polyoxyethylene sorbitan monolaurate (such as polysorbate 20), polyoxyethylene sorbitan monopalmitate (such as polysorbate 40), polyoxyethylene sorbitan monostearate (such as polysorbate 60), and polyoxyethylene monooleate (such as polysorbate 80).

In particular, from the viewpoint of promptly enhancing the effect of reducing curls and waves in hair, polyoxyethylene sorbitan monolaurate and polyoxyethylene sorbitan monopalmitate are preferable, and polyoxyethylene sorbitan monolaurate is more preferable.

Examples of polyoxyethylene alkyl ethers include polyoxyethylene(5) lauryl ether, polyoxyethylene(23) lauryl ether, polyoxyethylene(7) cetyl ether, polyoxyethylene(13) cetyl ether, polyoxyethylene(12) stearyl ether, and polyoxyethylene(13) oleyl ether.

In particular, from the viewpoint of promptly enhancing the effect of reducing curls and waves in hair, polyoxyethylene lauryl ether and polyoxyethylene cetyl ether are preferable, and polyoxyethylene lauryl ether is more preferable.

The content of one or more selected from the group consisting of these fatty acid polyoxyethylene sorbitan esters and polyoxyethylene alkyl ethers in the hair cleansing composition of the present invention is, from the viewpoint of promptly enhancing the effect of reducing curls and waves in hair, preferably 0.1 mass% or more, more preferably 0.2 mass% or more, and further more preferably 0.5 mass% or more, and is preferably 15 mass% or less, more preferably 12 mass% or less, and further more preferably 10 mass% or less. The content of one or more selected from the group consisting of these fatty acid polyoxyethylene sorbitan esters and polyoxyethylene alkyl ethers in the hair cleansing composition of the present invention is preferably from 0.1 to 15 mass%, more preferably from 0.2 to 12 mass%, and further more preferably from 0.5 to 10 mass%.

The hair cleansing composition of the present invention may further contain a basic amino acid. This enables the effect of reducing curls and waves in hair to be effectively and promptly exhibited. Examples of such components include arginine, lysine, histidine, and guanidine. In particular, arginine is preferable from the viewpoint of effectively and promptly enhancing the effect of reducing curls and waves in hair.

The content of the basic amino acid in the hair cleansing composition of the present invention is preferably 0.5 mass% or more and more preferably 1.0 mass% or more, and is preferably 15 mass% or less, more preferably 12 mass% or less, and further more preferably 10 mass% or less, from the viewpoint of effectively and promptly enhancing the effect of reducing curls and waves in hair. In addition, the content of the basic amino acid in the hair cleansing composition of the present invention is preferably from 0.5 to 15 mass%, more preferably from 1.0 to 12 mass%, and even more preferably from 1.0 to 10 mass%.

Preferably the hair cleansing composition of the present invention further contains water. Accordingly, the components in the hair cleansing composition can be favorably dispersed or dissolved, promoting penetration into hair. Examples of water include deionized water and distilled water.

The water content in the hair cleansing composition of the present invention is preferably 10 mass% or more, more preferably 30 mass% or more, and further more preferably 50 mass% or more, and is preferably 95 mass% or less. In addition, the water content in the hair cleansing composition of the present invention is preferably from 10 to 95 mass%, more preferably from 30 to 95 mass%, and further more preferably from 50 to 95 mass%.

Fatty acids or salts thereof are preferably restricted from being contained in the hair cleansing composition of the present invention from the viewpoint of avoiding a loss of storage stability while maintaining the superior effect of reducing curls and waves in hair. Examples of such fatty acids or salts thereof include fatty acids having 8 or more and 22 or less carbon atoms or salts thereof. Specifically, examples include decanoic acid, lauric acid, myristic acid, palmitic acid, palmitoleic acid, stearic acid, oleic acid, linoleic acid, linolenic acid, and salts thereof, and examples of salts of fatty acids include inorganic salts selected from the group consisting of sodium salts, potassium salts, magnesium salts, and ammonium salts, and organic amine salts such as monoethanolamine salts, diethanolamine salts, and triethanolamine salts.

The content of such fatty acids or salts thereof in the hair cleansing composition of the present invention is preferably 2.5 mass% or less, more preferably 1.5 mass% or less, and further more preferably 0.1 mass% or less.

Also, when the hair cleansing composition of the present invention contains a fatty acid or a salt thereof, the content of a fatty acid having 14 carbon atoms or a salt thereof in the total amount of the aforementioned fatty acid or a salt thereof is preferably less than 80 mass%, more preferably less than 50 mass%, and further more preferably less than 20 mass% from the viewpoint of storage stability.

The hair cleansing composition of the present invention may contain, in addition to the above components, components commonly used in hair cleansing agents as long as the effects of the present invention are not impaired. Examples of such components include surfactants excluding the above components, antioxidants, oils, anti-dandruffs, vitamins, bactericides, anti-inflammatory agents, preservatives, chelating agents, moisturizing agents, pearly pigments, ceramides, fragrances, UV absorbers, and pH adjusters.

The hair cleansing composition of the present invention has a pH of 1 or more and less than 5 at 25°C when formed into a 5 mass% aqueous solution. Accordingly, penetration of the components contained in the hair cleansing composition of the present invention into hair can be effectively and efficiently promoted.

The pH at 25°C of the hair cleansing composition of the present invention when formed into a 5 mass% aqueous solution is 1 or more, preferably 2 or more, and more preferably 3 or more, and is less than 5, preferably 4.5 or less, and more preferably 4 or less, from the viewpoint of effectively and efficiently promoting penetration of the components contained in the hair cleansing composition of the present invention into hair. The pH at 25°C when the hair cleansing composition of the present invention is formed into a 5 mass% aqueous solution is 1 or more and less than 5, preferably from 2 to 4.5, and more preferably from 3 to 4.

The pH at 25°C when the hair cleansing composition of the present invention is formed into a 5 mass% aqueous solution specifically means a value measured by the method described in the Examples.

The hair cleansing composition of the present invention is also extremely useful as a composition for suppressing curls and/or waves of hair.

To suppress curls and/or waves of hair with the hair cleansing composition of the present invention, specifically, for example, a method involving applying the hair cleansing composition of the present invention to hair and then rinsing the hair may be carried out.

More specifically, first the hair cleansing composition of the present invention may be placed on the palm, then the hands may be rubbed against each other to suitably create foam, and the composition may be applied to hair. From the viewpoint of favorably spreading the hair cleansing composition of the present invention over the hair, the hair is preferably untangled in advance with a tool such as a brush, and the hair is also preferably wetted with water in advance. The amount of the hair cleansing composition of the present invention to be applied to hair is preferably 0.2 g or more and 50 g or less per 100 g of hair per time.

After applying the hair cleansing composition of the present invention to hair, the hair may be washed with the palms or a tool such as a brush.

After applying the hair cleansing composition to hair, the time required until rinsing the hair is preferably 1 minute or longer, and may be 10 minutes or shorter or 5 minutes or shorter because the hair cleansing composition of the present invention can effectively and promptly reduce curls and waves in hair in a sufficient manner.

Regarding the above-described embodiments, the present invention further discloses the following hair cleansing compositions:

[1] A hair cleansing composition comprising the following components (A) and (B):

(A) 1 mass% or more and 8 mass% or less of an aromatic sulfonic acid or a salt thereof, and
(B) 4 mass% or more and 25 mass% or less of an internal olefin sulfonic acid having 16 carbon atoms or a salt thereof obtained by sulfonation of a raw material olefin having 16 carbon atoms and having an average double bond position of 3.9-position or more and 4.4-position or less,

wherein the hair cleansing composition has a pH at 25°C of 1 or more and less than 5 when formed into a 5 mass% aqueous solution.

[2] A hair cleansing composition comprising following components (A) and (B):

(A) 1 mass% or more and 6 mass% or less of an aromatic sulfonic acid or a salt thereof, and
(B) 4 mass% or more and 25 mass% or less of an internal olefin sulfonic acid having 16 carbon atoms or a salt thereof obtained by sulfonation of a raw material olefin having 16 carbon atoms and having an average double bond position of 3.9-position or more and 4.4-position or less,

wherein the hair cleansing composition has a mass ratio of a content of the component (A) to a content of the component (B), (A)/(B), of 0.08 or more and 1.5 or less, and has a pH at 25°C of 2 or more and less than 4.5 when formed into a 5 mass% aqueous solution.

[3] A hair cleansing composition comprising following components (A) and (B):

(A) 2 mass% or more and 4 mass% or less of an aromatic sulfonic acid or a salt thereof, and

(B) 4 mass% or more and 25 mass% or less of an internal olefin sulfonic acid having 16 carbon atoms or a salt thereof obtained by sulfonation of a raw material olefin having 16 carbon atoms and having an average double bond position of 3.9-position or more and 4.4-position or less,

wherein the hair cleansing composition has a mass ratio of a content of the component (A) to a content of the component (B), (A)/(B), of 0.13 or more and 1.0 or less, and has a pH at 25°C of 3 or more and less than 4 when formed into a 5 mass% aqueous solution.

[4] The hair cleansing composition according to any one of the above [1] to [3], wherein the component (A) is preferably one or more selected from the group consisting of benzenesulfonic acid or a salt thereof, naphthalenesulfonic acid or a salt thereof, azulenesulfonic acid or a salt thereof, and benzophenonesulfonic acid or a salt thereof, and the salt of the component (A) is a sodium salt, a potassium salt, a lithium salt, an aluminum salt, an ammonium salt ($NH_4^+$), or an organoammonium salt.

[5] The hair cleansing composition according to any one of the above [1] to [4], wherein the component (B) is preferably one or more selected from the group consisting of alkali metal salts, organic amine salts, and basic amino acid salts, more preferably one or more selected from the group consisting of a sodium salt, a potassium salt, an ammonium salt, and a 2-aminoethanol salt, further more preferably one or two selected from the group consisting of a sodium salt and a potassium salt, and further more preferably a sodium salt.

[6] The hair cleansing composition of any one of the above [1] to [5], wherein a content of the component (A) in the hair cleansing composition of the present invention is preferably 1.5 mass% or more and more preferably 2 mass% or more, and is preferably 6 mass% or less and more preferably 4 mass% or less.

[7] The hair cleansing composition according to any one of the above [1] to [6], wherein a content of the component (B) is preferably 20 mass% or less, more preferably 18 mass% or less, and further more preferably 16 mass% or less.

[8] The hair cleansing composition according to any one of the above [1] to [7], wherein the mass ratio of a content of the component (A) to a content of the component (B), (A)/(B), is preferably 0.05 or more, more preferably 0.08 or more, further more preferably 0.13 or more, and even more preferably 0.15 or more, and is preferably 2 or less, more preferably 1.5 or less, further more preferably 1.0 or less, and even more preferably 0.8 or less.

[9] The hair cleansing composition according to any one of the above [1] to [8], having a pH at 25°C of preferably 2 or more and more preferably 3 or more, and less than 5, preferably 4.5 or less, and more preferably 4 or less when formed into a 5 mass% aqueous solution.

[10] The hair cleansing composition according to any one of the above [1] to [9], further comprising one or more selected from the group consisting of fatty acid polyoxyethylene sorbitan esters and polyoxyethylene alkyl ethers.

[11] The hair cleansing composition according to the above [10], wherein a content of one or more selected from the group consisting of fatty acid polyoxyethylene sorbitan esters and polyoxyethylene alkyl ethers in the hair cleansing composition of the present invention is preferably 0.1 mass% or more, more preferably 0.2 mass% or more, and further more preferably 0.5 mass% or more, and is preferably 15 mass% or less, more preferably 12 mass% or less, and further more preferably 10 mass% or less.

[12] The hair cleansing composition according to any one of the above [1] to [11], further comprising a basic amino acid.

[13] The hair cleansing composition according to the above [12], wherein a content of the basic amino acid in the hair cleansing composition of the present invention is preferably 0.5 mass% or more and more preferably 1.0 mass% or more, and is preferably 15 mass% or less, more preferably 12 mass% or less, and further more preferably 10 mass% or less.

[14] A method for suppressing curls and/or waves of hair, comprising applying the hair cleansing composition according to any one of the above [1] to [12] to hair and then rinsing the hair.

[15] Use of the hair cleansing composition according to any one of the above [1] to [12] to suppress curls and/or waves of hair, wherein the hair cleansing composition is applied to the hair, and then the hair is rinsed.

Examples

The present invention will now be specifically described based on examples. Unless otherwise indicated in the table, the content of each component indicates mass%.

[Measurement methods for various physical properties]

(i) Method for measuring double bond position of raw material olefin

The double bond position of a raw material olefin was measured by gas chromatography (hereinafter, abbreviated as GC). Specifically, a dithiolated derivative of a raw material olefin was obtained by reaction with dimethyl disulfide, and then each component was separated by GC. As a result, the double bond positions of the raw material olefin were determined from the respective peak areas.

The apparatuses and analysis conditions used for measurement are as follows: GC apparatus (trade name: HP6890, manufactured by Hewlett-Packard Company); column (trade name: Ultra-Alloy-1HT capillary column 30 m × 250 $\mu$m × 0.15 $\mu$m, manufactured by Frontier Laboratories Ltd.); detector (hydrogen flame ionization detector (FID)); injection temperature: 300°C; detector temperature: 350°C; and flow rate of He: 4.6 mL/min.

(ii) Method for measuring content according to sulfonate group bonding position of sodium internal olefin sulfonate

Regarding sodium internal olefin sulfonate with an attached sulfonate group, each sodium internal olefin sulfonate content according to the sulfonate group bonding position was measured by high performance liquid chromatography/mass spectrometer (HPLC-MS). Specifically, hydroxy forms with a sulfonate group attached were separated by high performance liquid chromatography (HPLC) and were each applied to mass spectrometer (MS) for identification. As a result, each content was determined from the HPLC-MS peak areas.

The apparatuses and conditions used for measurement were as follows: HPLC apparatus "LD20ASXR" (manufactured by Shimadzu Corporation); column "ODS Hypersil (registered trademark)" (4.6 × 250 mm, particle size: 3 $\mu$m, manufactured by Thermo Fisher Scientific); sample preparation (1 000 times dilution with methanol); eluent A (10 mM ammonium acetate added water); eluent B (10 mM ammonium acetate added methacrylonitrile/water = 95/5 (v/v) solution); gradient (0 minutes (A/B = 60/40) -> 15.1 to 20 minutes (30/70) -> 20.1 to 30 minutes (60/40)); MS apparatus "LCMS-2020" (manufactured by Shimadzu Corporation); ESI detector (anion detection m/z: 321.10 (A) component having 16 or 18 carbon atoms); column temperature (40°C); flow rate (0.5 mL/min); and injection volume (5 $\mu$L).

(iii) Method for measuring mass ratio of hydroxy form/olefin form

The mass ratio of hydroxy form/olefin form of sodium internal olefin sulfonate was measured by HPLC-MS. Specifically, the hydroxy form and the olefin form were separated by HPLC and were respectively applied to MS for identification. As a result, the rate of each of them was determined from the HPLC-MS peak areas.

The apparatuses and conditions used for the measurement were as follows: HPLC apparatus (trade name: Agilent Technology 1100, manufactured by Agilent Technologies, Inc.); column (trade name: L-column ODS 4.6 × 150 mm, manufactured by Chemicals Evaluation and Research Institute, Japan); sample preparation (1 000 times dilution with methanol); eluent A (10 mM ammonium acetate added water); eluent B (10 mM ammonium acetate added methanol); gradient (0 minutes (A/B = 30%/70%) -> 10 minutes (30%/70%) -> 55 minutes (0%/100%) -> 65 minutes (0%/100%) -> 66 minutes (30%/70%) -> 75 minutes (30%/70%)); MS apparatus (trade name: Agilent Technology 1100 MS SL (G1946D), manufactured by Agilent Technologies, Inc.); and MS detection (anion detection m/z 60-1600, UV 240 nm).

(iv) Method for measuring content of raw material olefin

The content of unreacted raw material olefin in the sodium internal olefin sulfonate was measured by GC. Specifically, ethanol and petroleum ether were added to a sodium internal olefin sulfonate aqueous solution, and extraction was then performed to obtain olefin in the petroleum ether phase. As a result, the raw material olefin was quantitatively measured from the GC peak area.

The apparatuses and analysis conditions used for the measurement were as follows: GC apparatus (trade name: Agilent Technology 6850, manufactured by Agilent Technologies, Inc.); column (trade name: Ultra-Alloy-1HT capillary column 15 m × 250 $\mu$m × 0.15 $\mu$m, manufactured by Frontier Laboratories Ltd.); detector (hydrogen flame ionization detector (FID)); injection temperature: 300°C; detector temperature: 350°C; and He flow rate: 3.8 mL/min.

(v) Method for measuring content of inorganic compound

The contents of inorganic compounds were measured by potentiometric titration and neutralization titration. Specifically, the content of $Na_2SO_4$ was quantitatively measured by determining the sulfate radical ($SO_4^{2-}$) by potentiometric titration. The content of NaOH was quantitatively measured by neutralization titration with dilute hydrochloric acid.

(vi) Method for measuring pH of hair cleansing composition

The obtained hair cleansing compositions were each formed into a 5 mass% aqueous solution with water, thoroughly stirred, and then measured at 25°C with a pH meter (HM-30R manufactured by DKK-TOA Corporation).

[Manufacturing Example b1: manufacturing of raw material olefin b1 having 16 carbon atoms]

1-Hexadecanol (product name: KALCOL 6098, manufactured by Kao Corporation) 7 000 g (28.9 mol) and $\gamma$-alumina (manufactured by STREM Chemicals, Inc.) 350 g (5 mass% with respect to the raw material alcohol) as a solid acid catalyst were placed in a flask equipped with a stirrer, and reaction was performed by stirring at 280°C for 8 hours while circulating nitrogen (7 000 mL/min) in the system. The alcohol conversion rate after the completion of the reaction was 100%. The obtained crude alkene internal olefin was transferred to a distillation flask and was distilled at 136°C to 160°C/4.0 mmHg to obtain a raw material olefin a1 having 16 carbon atoms with an olefin purity of 100%. The double bond distribution of the obtained raw material olefin a1 was C1-position: 1.8 mass%, C2-position: 21.8 mass%, C3-position: 18.7 mass%, C4-position: 18.6 mass%, C5-position: 14.3 mass%, C6-position: 11.4 mass%, and the total of C7- and C8-positions: 13.6 mass%, and the average double bond position was 4.17.

[Manufacturing Example b2: manufacturing of raw material olefin b2 having 16 carbon atoms]

A raw material olefin b2 having 16 carbon atoms was obtained with an olefin purity of 100% as in Manufacturing Example b1 except that the reaction time was changed to 7.5 hours. The double bond distribution of the obtained raw material olefin b2 was C1-position: 2.4 mass%, C2-position: 23.2 mass%, C3-position: 18.7 mass%, C4-position: 18.2 mass%, C5-position: 13.9 mass%, C6-position: 11.2 mass%, and the total of C7- and the 8-positions: 12.4 mass%, and the average double bond position was 4.08.

[Manufacturing Example b3: manufacturing of raw material olefin b3 having 16 carbon atoms]

A raw material olefin b3 having 16 carbon atoms was obtained with an olefin purity of 100% as in Manufacturing Example b1 except that the reaction time was changed to 8.5 hours. The double bond distribution of the obtained raw material olefin b3 was C1-position: 2.3 mass%, C2-position: 20.7 mass%, C3-position: 16.8 mass%, C4-position: 17.5 mass%, C5-position: 14.7 mass%, C6-position: 12.9 mass%, and the total of C7- and 8-positions: 15.2 mass%, and the average double bond position was 4.28.

[Manufacturing Example b4: manufacturing of raw material olefin b4 having 16 carbon atoms]

A raw material olefin b4 having 16 carbon atoms was obtained with an olefin purity of 100% as in Manufacturing Example b1 except that the reaction time was changed to 6.5 hours. The double bond distribution of the obtained raw material olefin b4 was C1-position: 2.3 mass%, C2-position: 29.7 mass%, C3-position: 22.7 mass%, C4-position: 17.3 mass%, C5-position: 11.1 mass%, C6-position: 8.0 mass%, and the total of C7- and 8-positions: 9.0 mass%, and the average double bond position was 3.70.

[Manufacturing Example b5: manufacturing of raw material olefin b5 having 16 carbon atoms]

A raw material olefin b5 having 16 carbon atoms was obtained with an olefin purity of 100% as in Manufacturing Example b1 except that the reaction time was changed to 9.5 hours. The double bond distribution of the obtained raw material olefin b5 was C1-position: 0.9 mass%, C2-position: 19.2 mass%, C3-position: 16.1 mass%, C4-position: 15.7 mass%, C5-position: 16.6 mass%, C6-position: 13.2 mass%, and the total of C7- and 8-positions: 18.4 mass%, and the average double bond position was 4.50.

[Manufacturing Example b6: manufacturing of raw material olefin b6 having 18 carbon atoms]

1-Octadecanol (product name: KALCOL 8098, manufactured by Kao Corporation) 7 000 g (25.9 mol) and $\gamma$-alumina (manufactured by STREM Chemicals, Inc.) 700 g (10 mass% with respect to the raw material alcohol) as a solid acid catalyst were placed in a flask equipped with a stirrer, and reaction was performed by stirring at 280°C for 11 hours while circulating nitrogen (7 000 mL/min) in the system. The alcohol conversion rate after the completion of the reaction was 100%. The obtained crude alkene internal olefin was transferred to a distillation flask and was distilled at 148°C to 158°C/0.5 mmHg to obtain a raw material olefin b6 having 18 carbon atoms with an olefin purity of 100%. The double bond distribution of the obtained raw material olefin b6 was C1-position: 1.8 mass%, C2-position: 26.4 mass%, C3-position: 21.1 mass%, C4-position: 17.5 mass%, C5-position: 11.7 mass%, C6-position: 8.3 mass%, C7-position: 5.9 mass%, and the total of C8- and 9-positions: 7.4 mass%, and the average double bond position was 4.00.

Each of the physical property values of the obtained raw material olefins b1 to b6 are shown in Table 1.

[Table 1]

| Raw material olefin | | b1 | b2 | b3 | b4 | b5 | b6 |
|---|---|---|---|---|---|---|---|
| Number of carbon atoms | | 16 | 16 | 16 | 16 | 16 | 18 |
| Double bond distribution in raw material olefin (mass%) | 1-position | 1.8 | 2.4 | 2.3 | 2.3 | 0.9 | 1.8 |
| | 2-position | 21.8 | 23.2 | 20.7 | 29.7 | 19.2 | 26.4 |
| | 3-position | 18.7 | 18.7 | 16.8 | 22.7 | 16.1 | 21.1 |
| | 4-position | 18.6 | 18.2 | 17.5 | 17.3 | 15.7 | 17.5 |
| | 5-position | 14.3 | 13.9 | 14.7 | 11.1 | 16.6 | 11.7 |
| | 6-position | 11.4 | 11.2 | 12.9 | 8.0 | 13.2 | 8.3 |
| | 7-position | 6.8 | 6.2 | 7.6 | 4.5 | 9.2 | 5.9 |
| | 8-position | 6.8 | 6.2 | 7.6 | 4.5 | 9.2 | 3.7 |
| | 9-position | | | | | | 3.7 |
| | Total | 100.0 | 100.0 | 100.0 | 100.0 | 100.0 | 100.0 |
| Average double bond position (DBP) | | 4.17 | 4.08 | 4.28 | 3.70 | 4.50 | 4.00 |

[Manufacturing Example 1: manufacturing of sodium internal olefin sulfonate B1 having 16 carbon atoms]

The raw material olefin b1 obtained in Manufacturing Example b1 was put in a thin film sulfonation reactor having an outer jacket, and sulfonation reaction was performed using a sulfur trioxide gas under the condition of allowing cooling water of 10°C to pass through the reactor outer jacket. The molar ratio of SOs/internal olefin during the sulfonation reaction was set to 1.01. The obtained sulfonated product was mixed with an alkali aqueous solution prepared with sodium hydroxide (alkali agent) in an amount of 1.04 times mol per mol of the theoretical acid value, and neutralization was performed by a continuous method at 30°C for 1 hour. The obtained neutralized product was heated in an autoclave at 170°C for 1 hour for hydrolysis to obtain sodium internal olefin sulfonate B1 having 16 carbon atoms. The content of the raw material olefin contained in the obtained sodium internal olefin sulfonate B1 having 16 carbon atoms was 0.4 mass%, and the content of the inorganic compound was 0.39 mass%.

[Manufacturing Example 2: manufacturing of sodium internal olefin sulfonate B2 having 16 carbon atoms]

Sodium internal olefin sulfonate B2 having 16 carbon atoms was obtained as in Manufacturing Example 1 except that the raw material olefin b2 obtained in Manufacturing Example b2 was used as the raw material olefin. The content of the raw material olefin contained in the obtained sodium internal olefin sulfonate B2 having 16 carbon atoms was 0.7 mass%, and the content of the inorganic compound was 0.49 mass%.

[Manufacturing Example 3: manufacturing of sodium internal olefin sulfonate B3 having 16 carbon atoms]

Sodium internal olefin sulfonate B3 having 16 carbon atoms was obtained as in Manufacturing Example 1 except that the raw material olefin b3 obtained in Manufacturing Example b3 was used as the raw material olefin. The content of the raw material olefin contained in the obtained sodium internal olefin sulfonate B3 having 16 carbon atoms was 0.5 mass%, and the content of the inorganic compound was 0.54 mass%.

[Manufacturing Example 4: manufacturing of sodium internal olefin sulfonate B4 having 16 carbon atoms]

Sodium internal olefin sulfonate B4 having 16 carbon atoms was obtained as in Manufacturing Example 1 except that the raw material olefin b4 obtained in Manufacturing Example b4 was used as the raw material olefin. The content of the raw material olefin contained in the obtained sodium internal olefin sulfonate B4 having 16 carbon atoms was 0.4 mass%, and the content of the inorganic compound was 0.41 mass%.

[Manufacturing Example 5: manufacturing of sodium internal olefin sulfonate B5 having 16 carbon atoms]

Sodium internal olefin sulfonate B5 having 16 carbon atoms was obtained as in Manufacturing Example 1 except that the raw material olefin b5 obtained in Manufacturing Example b5 was used as the raw material olefin. The content of the raw material olefin contained in the obtained sodium internal olefin sulfonate B5 having 16 carbon atoms was 0.3 mass%, and the content of the inorganic compound was 0.43 mass%.

[Manufacturing Example 6: manufacturing of sodium internal olefin sulfonate B6 having 18 carbon atoms]

Sodium internal olefin sulfonate B6 having 18 carbon atoms was obtained as in Manufacturing Example 1 except that the raw material olefin b6 obtained in Manufacturing Example b6 was used as the raw material olefin. The content of the raw material olefin contained in the obtained sodium internal olefin sulfonate B6 having 18 carbon atoms was 0.5 mass%, and the content of the inorganic compound was 0.45 mass%.

Each of the physical property values of the obtained sodium internal olefin sulfonates B1 to B6 are shown in Table 2.

[Table 2]

| Sodium internal olefin sulfonate | | B1 | B2 | B3 | B4 | B5 | B6 |
|---|---|---|---|---|---|---|---|
| Raw material olefin | | b1 | b2 | b3 | b4 | b5 | b6 |
| Distribution of sulfonate group (mass%) | 1-position | 2.0 | 1.2 | 1.6 | 1.6 | 0.8 | 1.4 |
| | 2-position | 24.8 | 18.7 | 17.5 | 18.4 | 13.9 | 20.7 |
| | 3-position | 19.1 | 16.1 | 15.7 | 15.2 | 12.1 | 17.4 |
| | 4-position | 22.0 | 19.9 | 20.3 | 19.3 | 18.1 | 21.0 |
| | 5- to 9-positions | 32.1 | 44.2 | 45.0 | 45.5 | 55.0 | 39.6 |
| | Total | 100 | 100 | 100 | 100 | 100 | 100 |
| Hydroxy form | | 83.9 | 84.2 | 83.8 | 84.0 | 84.5 | 83.8 |
| Olefin form | | 16.1 | 15.8 | 16.2 | 16.0 | 15.5 | 16.2 |

[Examples 1 to 12, Comparative Examples 1 to 9]

Hair cleansing compositions having the compositions shown in Tables 3 to 4 were prepared by a usual method using the obtained sodium internal olefin sulfonates B1 to B6 that were suitably obtained as necessary. Specifically, a component (A), a component (B), an appropriate amount of water, and other components as needed were placed in a beaker and were heated to 60 to 80°C, mixed, and cooled to room temperature. Subsequently, water was replenished to obtain each hair cleansing composition.

Using the obtained hair cleansing compositions, the following operations 1) to 7) that take in consideration the short-time haircare activities repetitively undertaken on a daily basis were carried out, and evaluations were made on the effect of reducing curls and waves in hair, the volume of foam created during application, and the degree of non-friction of hair during rinsing.

The results are shown in Tables 3 and 4.

<<Curl reducing rate»

1) Curled Caucasian hair (manufactured by Kerling International Haarfabrik, European-natural hair remis curly 30 to 35 cm) was placed on a flat plate and cut out at the points of inflection as viewed from above (hereafter referred to as C-curl hair), and this was immersed in deionized water for 20 minutes and left to stand still at 20°C and 50% RH for at least 24 hours.

2) The C-curl hair was placed on 1 mm graph paper and photographed from above. Using the obtained image and assuming that the C-curl hair was a circular arc, the diameter $D_0$ of a circle inscribed in the circular arc was measured.

3) Next, the C-curl hair was immersed in a hair cleansing composition at 40°C for 5 minutes, and then immersed in deionized water for 1 minute.

4) The operation of the above 3) was repeated five times.

5) Next, the obtained C-curl hair was immersed in deionized water for 20 minutes, and then left to stand at 20°C and 50% RH for at least 24 hours.

6) Next, assuming that the obtained C-curl hair was a circular arc, the diameter D of a circle inscribed in the circular arc was measured, and the value of diameter D/diameter $D_0$ was calculated.

7) The operations of the above 1) to 6) were performed on five separately cut pieces of the C-curl hair, the average value thereof was calculated as a curl reducing rate and used as an index for evaluating the effect of reducing curls and waves in hair.

Note that when the value of the obtained curl reducing rate is 1.10 or more, a hair cleansing composition can be judged as having a superior effect of reducing curls and waves in hair even when the duration of use per time is relatively short.

<<Volume of foam created during application>>

After 20 g of tress (about from 15 to 20 cm) of a Japanese woman was wetted with water, 1 g of the obtained hair cleansing composition was applied to the hand, and shampooing was carried out by moving the hand such that the hair cleansing composition was sufficiently spread over the hair. The volume of foam created at this time was evaluated by a panel of three experts according to the following criteria, and the average value thereof was determined.

- Evaluation criteria

5: Abundant
4: Relatively abundant
3: Neither abundant nor scarce
2: Relatively scarce
1: Scarce

<<Degree of non-friction of hair during rinsing>>

After 20 g of tress (about from 15 to 20 cm) of a Japanese woman was sufficiently wetted with water, 1 g of the obtained hair cleansing composition was applied to the hand, and shampooing was carried out by moving the hand such that the hair cleansing composition was sufficiently spread over the hair. Thereafter, the degree of non-friction of hair when hand-combed during rinsing was evaluated by a panel of three experts according to the following criteria, and the average value thereof was determined.

- Evaluation criteria

5: Hair is non-frictional until end of rinsing
4: Hair becomes frictional after a lapse of about 3/4 of the time between the start and the end of rinsing
3: Hair becomes frictional after a lapse of about 1/2 of the time between the start and the end of rinsing
2: Hair becomes frictional after a lapse of about 1/4 of the time between the start and the end of rinsing
1: Hair is frictional from the start of rinsing

[Table 3]

| | | Example 1 | Example 2 | Example 3 | Example 4 | Example 5 | Example 6 | Example 7 | Example 8 | Comp. Example 1 | Comp. Example 2 | Comp. Example 3 | Comp. Example 4 | Comp. Example 5 | Comp. Example 6 | Comp. Example 7 |
|---|---|---|---|---|---|---|---|---|---|---|---|---|---|---|---|---|
| (A) | p-Toluenesulfonate*1 | 3.0 | | 3.0 | 2.0 | 2.0 | 2.0 | 3.0 | 3.0 | 3.0 | 3.0 | 3.0 | 0.9 | 3.0 | 3.0 | 3.0 |
| | Sodium 2-Naphthalenesulfonate*2 | | 3.0 | | | | | | | | | | | | | |
| (B) | Sodium internal olefin sulfonate B1 (DBP4.17, C16) | 16.0 | 16.0 | 16.0 | 16.0 | 16.0 | 16.0 | | | | | 16.0 | 16.0 | | | |
| | Sodium internal olefin sulfonate B2 (DBP4.08, C16) | | | | | | | 16.0 | | | | | | | | |
| | Sodium internal olefin sulfonate B3 (DBP4.28, C16) | | | | | | | | 16.0 | | | | | | | |
| | Sodium internal olefin sulfonate B4 (DBP3.70, C16) | | | | | | | | | | | | | 16.0 | | |
| | Sodium internal olefin sulfonate B5 (DBP4.50, C16) | | | | | | | | | | | | | | 16.0 | |
| | Sodium internal olefin sulfonate B6 (DBP4.00, C18) | | | | | | | | | | | | | | | 16.0 |
| | Ammonium polyoxyethylene(1) lauryl ether sulfate*3 | | | | | | | | | 16.0 | | | | | | |
| | Sodium α-olefinsulfonate*4 | | | | | 3.0 | | | | | 16.0 | | | | | |
| | Polyoxyethylene sorbitan monolaurate (20E.O.)*5 | | | | | | | | | | | | | | | |

(continued)

| | Exam-ple 1 | Exam-ple 2 | Exam-ple 3 | Exam-ple 4 | Exam-ple 5 | Exam-ple 6 | Exam-ple 7 | Exam-ple 8 | Comp. Exam-ple 1 | Comp. Exam-ple 2 | Comp. Exam-ple 3 | Comp. Exam-ple 4 | Comp. Exam-ple 5 | Comp. Exam-ple 6 | Comp. Exam-ple 7 |
|---|---|---|---|---|---|---|---|---|---|---|---|---|---|---|---|
| Arginine[*6] | | | | | | 3.0 | | | | | | | | | |
| Citric acid[*7] | | Suitable amount[*9] | | | | Suitable amount[*9] | | | | | | | | | |
| Potassium hydroxide[*8] | Suitable amount[*9] | | Suitable amount[*9] | Suitable amount[*9] | Suitable amount[*9] | | Suitable amount[*9] | Suitable amount[*9] | Suitable amount[*9] | Suitable amount[*9] | Suitable amount[*9] | Suitable amount[*9] | Suitable amount[*9] | Suitable amount[*9] | Suitable amount[*9] |
| Deionized water | Balance | Balance | Balance | Balance | Balance | Balance | Balance | Balance | Balance | Balance | Balance | Balance | Balance | Balance | Balance |
| Total | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 |
| pH at 25°C when formed into 5 mass% aqueous solution | 3.6 | 3.4 | 4.6 | 3.7 | 3.5 | 3.7 | 3.7 | 3.7 | 3.8 | 3.6 | 5.6 | 3.6 | 3.6 | 3.5 | 3.7 |
| Total amount of (A) and (B) | 19.0 | 19 | 19 | 18 | 18 | 18 | 19.0 | 19.0 | 3 | 3 | 19 | 16.9 | 3 | 3 | 3 |
| (A)/(B) | 0.19 | 0.19 | 0.19 | 0.13 | 0.13 | 0.13 | 0.19 | 0.19 | - | - | 0.19 | 0.06 | - | - | - |
| Curl reducing rate | 1.16 | 1.11 | 1.12 | 1.12 | 1.14 | 1.14 | 1.14 | 1.11 | 1.09 | 1.06 | 1.04 | 1.09 | 1.05 | 1.05 | 1.08 |
| Volume of foam during application | 4.3 | 5 | 4.3 | 4.3 | 5 | 4.7 | 4.3 | 4.3 | 4.3 | 3.7 | 4.7 | 4 | 4.3 | 4.3 | 2.3 |

(continued)

| | Example 1 | Example 2 | Example 3 | Example 4 | Example 5 | Example 6 | Example 7 | Example 8 | Comp. Example 1 | Comp. Example 2 | Comp. Example 3 | Comp. Example 4 | Comp. Example 5 | Comp. Example 6 | Comp. Example 7 |
|---|---|---|---|---|---|---|---|---|---|---|---|---|---|---|---|
| Extent of smoothness of hair during rinsing | 3.7 | 3.3 | 3.7 | 4 | 4.3 | 3.7 | 3.7 | 3.3 | 4.3 | 3.3 | 3.7 | 3.3 | 3.3 | 3 | 3.7 |

*1: PTS70% solution, manufactured by Konan Chemical Manufacturing Co., Ltd.

*2: Sodium β-naphthalenesulfonic acid (B-NAPH.S.A.S.S), manufactured by Sugai Chemical Industry Co., Ltd.

*3: Emal 125A, manufactured by Kao Corporation *4: K Lipolan PJ-400CJ, manufactured by Lion Specialty Chemicals Co., Ltd.

*5: Rheodol TW-L120, manufactured by Kao Corporation *6: L-Arginine C grade, manufactured by Ajinomoto Co., Inc.

*7: Citric acid (50%), manufactured by Showa Kako Corp.

*8: Liquid caustic potassium (48%), manufactured by AGC Inc.

*9: Amount required to form 5 mass% aqueous solution for pH value at 25°C

[Table 4]

| | | Comp. Example 4 | Example 9 | Example 4 | Example 1 | Example 10 | Example 11 | Example 12 | Comp. Example 8 | Comp. Example 9 |
|---|---|---|---|---|---|---|---|---|---|---|
| (A) | p-Toluenesulfonic acid*1 | 0.9 | 1.2 | 2.0 | 3.0 | 7.0 | 4.0 | 7.0 | 10.0 | 8.0 |
| (B) | Sodium internal olefin sulfonate B1 | 16.0 | 20.0 | 16.0 | 16.0 | 12.0 | 6.0 | 6.0 | 4.0 | 2.0 |
| | Potassium hydroxide*8 | Suitable amount*9 | Suitable amount*9 | Suitable amount*9 | Suitable amount*9 | Suitable amount*9 | Suitable amount*9 | Suitable amount*9 | Suitable amount*9 | Suitable amount*9 |
| | Deionized water | Balance | Balance | Balance | Balance | Balance | Balance | Balance | Balance | Balance |
| | Total | 100.0 | 100.0 | 100.0 | 100.0 | 100.0 | 100.0 | 100.0 | 100.0 | 100.0 |
| | pH at 25°C when formed into 5 mass% aqueous solution | 3.6 | 3.5 | 3.7 | 3.6 | 3.2 | 3.5 | 3.2 | 3.7 | 3.6 |
| | Total amount of (A) and (B) | 16.9 | 21.2 | 18 | 19.0 | 19 | 10 | 13 | 14 | 10 |
| | (A)/(B) | 0.06 | 0.06 | 0.13 | 0.19 | 0.58 | 0.67 | 1.17 | 2.50 | 4.00 |
| | Curl reducing rate | 1.09 | 1.12 | 1.12 | 1.16 | 1.15 | 1.14 | 1.13 | 1.11 | 1.07 |
| | Volume of foam when applied | 4 | 4.7 | 4.3 | 4.3 | 4.3 | 3.7 | 3.7 | 2.3 | 1 |
| | Extent of smoothness of hair during rinsing | 3.3 | 3.7 | 4 | 3.7 | 3.7 | 3.3 | 3.7 | 2 | 1.3 |

*1, 8, 9: Same as Table 3

**Claims**

1. A hair cleansing composition comprising the following components (A) and (B):

   (A) 1 mass% or more and 8 mass% or less of an aromatic sulfonic acid or a salt thereof, and
   (B) 4 mass% or more and 25 mass% or less of an internal olefin sulfonic acid having 16 carbon atoms or a salt thereof obtained by sulfonation of a raw material olefin having 16 carbon atoms and having an average double bond position of 3.9-position or more and 4.4-position or less,

   wherein the hair cleansing composition has a pH at 25°C of 1 or more and less than 5 when formed into a 5 mass% aqueous solution.

2. The hair cleansing composition according to claim 1, wherein a mass ratio of a content of the component (A) to a content of the component (B), (A)/(B), is 0.05 or more and 2 or less.

3. The hair cleansing composition according to claim 1 or 2, wherein a content of an internal olefin sulfonic acid or a salt thereof including a sulfonate group at the 1-position or more and the 4-position or less in the component (B) is 40 mass% or more and 75 mass% or less.

4. The hair cleansing composition according to any one of claims 1 to 3, wherein a content of an internal olefin sulfonic acid or a salt thereof including a sulfonate group at the 2-position in the component (B) is 10 mass% or more and 35 mass% or less.

5. The hair cleansing composition according to any one of claims 1 to 4, wherein a content of an internal olefin sulfonic acid or a salt thereof including a sulfonate group at the 3-position in the component (B) is 5 mass% or more and 30 mass% or less.

6. The hair cleansing composition according to any one of claims 1 to 5, wherein a mass ratio of a content of a hydroxy form of the internal olefin sulfonic acid or a salt thereof to a content of an olefin form of the internal olefin sulfonic acid or a salt thereof, (hydroxy form/olefin form), in the component (B) is from 50/50 to 100/0.

7. The hair cleansing composition according to any one of claims 1 to 6, which is for suppressing curls and/or waves of hair.

## INTERNATIONAL SEARCH REPORT

| | International application No. |
|---|---|
| | **PCT/JP2023/004500** |

**A.    CLASSIFICATION OF SUBJECT MATTER**

*A61Q 5/02*(2006.01)i; *A61K 8/46*(2006.01)i
FI:    A61K8/46; A61Q5/02

According to International Patent Classification (IPC) or to both national classification and IPC

**B.    FIELDS SEARCHED**

Minimum documentation searched (classification system followed by classification symbols)

A61Q5/02; A61K8/46

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| Y | JP 2015-027977 A (KAO CORP.) 12 February 2015 (2015-02-12)<br>claims, paragraphs [0009], [0012], [0169], [0181], [0188], [0207], [0208] | 1-7 |
| Y | JP 2021-011452 A (KAO CORP.) 04 February 2021 (2021-02-04)<br>paragraphs [0008], [0009], [0030], [0031] | 1-7 |
| A | JP 2015-27976 A (KAO CORP.) 12 February 2015 (2015-02-12)<br>entire text | 1-7 |

☐ Further documents are listed in the continuation of Box C.    ☑ See patent family annex.

| | |
|---|---|
| *      Special categories of cited documents:<br>"A"    document defining the general state of the art which is not considered to be of particular relevance<br>"E"    earlier application or patent but published on or after the international filing date<br>"L"    document which may throw doubts on priority claim(s) or which is cited to establish the publication date of another citation or other special reason (as specified)<br>"O"    document referring to an oral disclosure, use, exhibition or other means<br>"P"    document published prior to the international filing date but later than the priority date claimed | "T"    later document published after the international filing date or priority date and not in conflict with the application but cited to understand the principle or theory underlying the invention<br>"X"    document of particular relevance; the claimed invention cannot be considered novel or cannot be considered to involve an inventive step when the document is taken alone<br>"Y"    document of particular relevance; the claimed invention cannot be considered to involve an inventive step when the document is combined with one or more other such documents, such combination being obvious to a person skilled in the art<br>"&"    document member of the same patent family |

| Date of the actual completion of the international search | Date of mailing of the international search report |
|---|---|
| **10 April 2023** | **25 April 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/004500**

| Patent document cited in search report | | | Publication date (day/month/year) | Patent family member(s) | | | Publication date (day/month/year) |
|---|---|---|---|---|---|---|---|
| JP | 2015-027977 | A | 12 February 2015 | US | 2014/0079660 | A1 | |
| | | | | claims, paragraphs [0014], [0022], [0059], [0066], [0087], [0121], [0126], [0134], [0160], [0161], table 1 | | | |
| | | | | WO | 2014/046300 | A2 | |
| JP | 2021-011452 | A | 04 February 2021 | US | 2022/0280404 | A1 | |
| | | | | paragraphs [0015], [0029], [0030] | | | |
| | | | | WO | 2021/006143 | A1 | |
| JP | 2015-27976 | A | 12 February 2015 | US | 2014/0076344 | A1 | |
| | | | | entire text | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019505546 A

- JP 2019073443 A

**Non-patent literature cited in the description**

- *J. Am. Oil Chem. Soc.*, 1992, vol. 69, 39